# EUROPÄISCHE PATENTSCHRIFT

(11) **EP 2 697 190 B1**
(45) Veröffentlichungstag und Bekanntmachung des Hinweises auf die Patenterteilung: **13.05.2015**
(21) Anmeldenummer: 12708334.3
(22) Anmeldetag: 13.03.2012
(51) Int. Cl.: C07C 227/18, C07C 229/12

(54) **VERFAHREN ZUR HERSTELLUNG VON AMINOPOLYCARBOXYLATEN AUSGEHEND VON AMINOSÄUREN**
METHOD FOR PRODUCING AMINO-POLYCARBOXYLATES FROM AMINO ACIDS
PROCÉDÉ DE PRODUCTION D'AMINOPOLYCARBOXYLATES DÉRIVÉS D'ACIDES AMINÉS

(30) Priorität: 12.04.2011 EP 11162091
(43) Veröffentlichungstag der Anmeldung: 19.02.2014
(73) Patentinhaber: BASF SE, 67056 Ludwigshafen (DE)
(72) Erfinder: BAUMANN, Robert, 68529 Mannheim (DE); BIEL, Markus, Christian, 68159 Mannheim (DE); FRANZKE, Axel, 68161 Mannheim (DE); HEIDENFELDER, Thomas, Randolph, NJ 07869 (US); KLINGELHOEFER, Paul, 68165 Mannheim (DE); OFTRING, Alfred, 67098 Bad Dürkheim (DE)
(86) Internationale Anmeldenummer: PCT/EP2012/054349
(87) Internationale Veröffentlichungsnummer: WO 2012/139842

(56) Entgegenhaltungen:
- EP-A1- 0 506 973
- WO-A1-2006/120129
- US-A1- 2011 257 431
- PASCAL M L: "N 89.-SYNTHESE DE MORPHOLONES-2, DES HYDROXYAMINOACIDES CORRESPONDANT ET DE LEURS CHELATES CUIVRIQUES, A PARTIR DES EPOXYDES-1,2 ET DES SELS DE SODIUM D'ALPHA-AMINOACIDES", BULLETIN DE LA SOCIETE CHIMIQUE DE FRANCE, SOCIETE FRANCAISE DE CHIMIE. PARIS, FRANCE, 1. Januar 1960 (1960-01-01), Seiten 435-442, XP000960955, ISSN: 0037-8968

## Beschreibung

Die Erfindung betrifft ein Verfahren zur Herstellung von Aminopolycarboxylaten ausgehend von Aminosäuren in zwei Verfahrensschritten, wobei in einem ersten Verfahrensschritt die Aminosäure mit Ethylenoxid zu einem Zwischenproduktgemisch, enthaltend das entsprechende Dialkanolamin, umgesetzt wird. Dieses Zwischenproduktgemisch wird anschließend katalytisch unter Einsatz einer Base zum entsprechenden Aminopolycarboxylat umgesetzt.

Die Ethoxylierung von Aminosäuren in wässrigem Medium wurde bisher nur für wenige Verbindungen beschrieben, z. B. Alanin, Phenylalanin, Asparaginsäure und Glutaminsäure, wobei die Säurefunktionen der jeweiligen Derivate üblicherweise mit Natronlauge vollständig deprotoniert und somit in das entsprechende Natriumcarboxylat überführt wurden (vgl. Arch. Pharm. (Weinheim) 1992, 325, 709, Bull. Soc. Chim. France 1960, 435 und EP 1 086 944).

Die oxidative Dehydrierung von Aminoalkoholen mit Alkalihydroxiden wird üblicherweise unter Druck und bei Temperaturen von 140 bis 220 °C unter Verwendung von Kupferkatalysatoren durchgeführt. Die Katalysatoren bestehen z. B. aus dotiertem oder undotiertem Raney-Kupfer (z. B. in WO 00/066539 beschrieben). Als Dotierstoffe werden in der Regel ein oder mehrere Metalle, wie z. B. Pt, Fe, Cr, Mo, V, Bi, Sn, Sb, Pb, Ge oder Ag verwendet.

In anderen Beispielen wird Kupfer direkt oder über Ankermetalle (z. B. Os, Ir, Rh, Pt, Pd) auf alkalistabile Träger aufgebracht (z. B. WO 98/50150). Auch Kupferfällkatalysatoren mit weiteren Metalloxiden wurden beschrieben (z. B. WO 03/051513 (Cu, Fe), EP 0 506 973 (Cu, Zr, Ca)). Vereinzelt ist über die Umsetzung an Edelmetallsystemen berichtet worden (z. B. EP 0 201 957).

WO2006120129 offenbart ein Verfahren zur Herstellung von Methylglycin-N,N-diessigsäuretrialkalimetallsalz durch alkalische Hydrolyse von Methylglycindiacetonitril.

Ein Problem bei der Herstellung insbesondere von Komplexbildnern wie MGDA (Methylglycindiessigsäure) oder GLDA (Glutaminsäurediessigsäure) und deren Salzen aus den zugehörigen Aminosäuren wie Alanin oder Glutaminsäure besteht darin, dass bei einer konventionellen Durchführung der beiden Verfahrensschritte relativ hohe Anteile an Nebenprodukten mit geringerer - Wirksamkeit oder sogar gesundheitsschädlichen Effekten erhalten werden.

Eine Aufreinigung durch gängige Trennverfahren ist nicht möglich, da Aminopolycarboxylate Salze sind, praktisch keinen Dampfdruck haben und daher nicht destilliert werden können. Durch Sprühtrocknung können nur Komponenten des Produktgemisches abgetrennt werden, die einen hinreichend hohen Dampfdruck aufweisen. Die meisten Nebenprodukte sind jedoch ebenfalls Salze und können auf diese Weise somit nicht entfernt werden. Auch das Zwischenprodukt, das nach dem ersten Verfahrensschritt, der Ethoxylierung, erhalten wird, liegt als Salz vor, d. h. auch die Aufreinigung des Zwischenproduktgemisches aus dem ersten Verfahrensschritt ist sehr schwierig und mit den oben genannten Problemen behaftet.

Es war daher Aufgabe der Erfindung, ein Verfahren zur Herstellung von Aminopolycarboxylaten ausgehend von Aminosäuren in zwei Verfahrensschritten, d. h. einer Ethoxylierung der Aminosäure zum Dialkanolamin und einer katalytischen oxidativen Dehydrierung des Dialkanolamins zum Aminopolycarboxylat zur Verfügung zu stellen, durch dessen Anwendung ein Produkt erhalten wird, das auch direkt ohne weitere Aufreinigung einen hohen Reinheitsgrad aufweist. Dies ist gleichbedeutend mit einer hohen Ausbeute an Aminopolycarboxylat über zwei Stufen ausgehend von der Aminosäure, wobei diese Ausbeute mindestens 91 %, bevorzugt mindestens 94 % sein sollte. Es soll somit ein technisch einfaches Verfahren zur Verfügung gestellt werden, das die Umsetzung von Aminosäuren zu den entsprechenden Aminopolycarboxylaten mit hohem Umsatz und hoher Selektivität gewährleistet, wobei die Aminopolycarboxylate, die durch die chemische Umsetzung erhalten werden, bereits direkt, ohne weitere Aufreinigung, einen ausreichend hohen Reinheitsgrad aufweisen, um als Verkaufsprodukt geeignet zu sein.

Diese Aufgabe wird überraschend gelöst durch ein Verfahren zur Herstellung von Aminopolycarboxylaten ausgehend von einer Aminosäure, die in einem
- ersten Verfahrensschritt mit Ethylenoxid zu einem Zwischenproduktgemisch, enthaltend das entsprechende Dialkanolamin, umgesetzt wird, und danach das Zwischenproduktgemisch in einem
- zweiten Verfahrensschritt katalytisch unter Einsatz einer Base zum entsprechenden Aminopolycarboxylat umgesetzt wird, das dadurch gekennzeichnet ist, dass
- die Aminosäure vor der Umsetzung mit Ethylenoxid im ersten Verfahrensschritt einer Teilneutralisation mit 0,70 bis 0,99 Äquivalenten Base pro Säuregruppe zugeführt wird oder dass im ersten Verfahrensschritt eine bereits mit 0,70 bis 0,99 Äquivalenten Base pro Säuregruppe teilneutralisierte Aminosäure eingesetzt wird.

Das vorliegende Verfahren betrifft eine zweistufige Umsetzung von Aminosäuren zu den entsprechenden Aminopolycarboxylaten nach der folgenden Reaktionssequenz 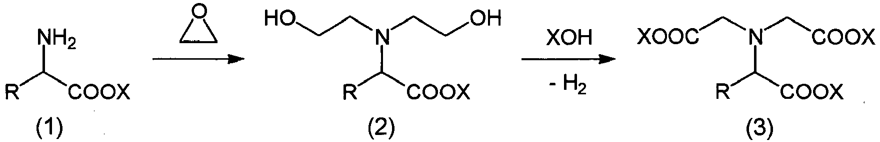 R = Alkyl, Alkenyl, Alkinyl, Aryl, Aralkyl, Hydroxyalkyl, Hydroxyaralkyl, Alkylencarboxyl, Alkylensulfonat und
X = Alkali- und/ oder Erdalkalimetall und teilweise Wasserstoff bei (1) und (2).

Nicht neutralisierte Aminosäuren (X = H in (1)) reagieren nur sehr schleppend mit Ethylenoxid, da der Stickstoff weitestgehend protoniert und daher nicht reaktiv ist. Deswegen ist es üblich, die als Edukte in einer Ethoxylierung eingesetzten Aminosäuren vollständig zu deprotonieren (X = Alkali- und/ oder Erdalkalimetall in (1)). In dem vorliegenden erfindungsgemäßen Verfahren wird die Aminosäure hingegen teilneutralisiert (X = Alkali- und/ oder Erdalkalimetall und teilweise Wasserstoff in (1)) eingesetzt. Dies bedeutet, dass weniger als 1,00 Äquivalente Base pro Säuregruppe verwendet werden. Als Säuregruppen werden vorliegend saure Funktionalitäten wie Carbonsäure- und Sulfonsäuregruppen, aber auch aus einer Protonierung von basischen Stickstoffatomen hervorgegangene Gruppen wie Ammoniumsalze bezeichnet.

Bei der Umsetzung im ersten Verfahrensschritt entstehen neben dem erwünschten Zwischenprodukt, dem entsprechenden Dialkanolamin, als weitere Hauptnebenprodukte, die sich ebenfalls von der Aminosäure ableiten, Monoalkanolamin, aus einer Veretherung des Dialkanolamins hervorgehendes Monoetherdialkanolamin und Trialkanolammoniumsalz.

Es wurde gefunden, dass der relative Anteil der im ersten Verfahrensschritt anfallenden Nebenkomponenten in hohem Maße vom Neutralisationsgrad der als Edukt eingesetzten Aminosäure abhängt. Beim Einsatz von stöchiometrischen Mengen an Base, d. h. einem 100-%igen Neutralisationsgrad der Aminosäure, entstehen die obigen drei neben dem Dialkanolamin anfallenden Nebenprodukte in vergleichbaren Mengen. Beim Einsatz von unterstöchiometrischen Mengen an Base bei ansonsten unveränderten Bedingungen der Ethoxylierung steigt der relative Anteil des Trialkanolammoniumsalzes im Nebenproduktspektrum deutlich an. Bei einem überstöchiometrischen Einsatz an Base verschwindet dagegen das Trialkanolammoniumsalz weitestgehend, wobei gleichzeitig der Gehalt der anderen Nebenkomponenten steigt.

Die Nebenprodukte Monoalkanolamin und Monoetherdialkanolamin sind unerwünscht, da sie in der zweiten Verfahrensstufe, der Umsetzung zu den jeweiligen Aminopolycarboxylaten, als Komplexbildner weitgehend inaktive Komponenten liefern. Das Trialkanolammoniumsalz ist dagegen vorteilhaft, da es sich unter den Reaktionsbedingungen des zweiten Verfahrensschrittes in das erwünschte Zwischenprodukt Dialkanolamin zersetzt, welches dann zusätzliches Wertprodukt bildet. Dieser Vorgang führt somit zu einer Ausbeutesteigerung.

Als Aminopolycarboxylate werden vorliegend Aminocarboxylate mit drei oder vier deprotonierten Carbonsäuregruppen bezeichnet. Aminopolycarboxylate mit drei deprotonierten Carbonsäuregruppen sind insbesondere Salze der Methylglycindiessigsäure, Aminopolycarboxylate mit vier deprotonierten Carbonsäuregruppen sind insbesondere Salze der Glutaminsäurediessigsäure. Aufgrund der obigen Struktur können die Aminopolycarboxylate vorteilhaft als Komplexbildner eingesetzt werden.

Die Aminosäure ist vorteilhaft ausgewählt aus der Gruppe der Aminosäuren der Formel (1) 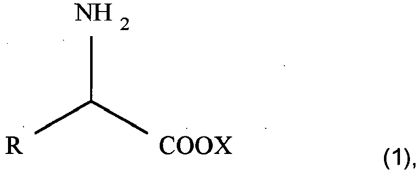 wobei
- R: ein Alkyl-, Alkenyl-, Alkinyl-, Aryl-, Aralkyl-, Hydroxyalkyl-, Hydroxyaralkyl-, Alkylencarboxyl- oder ein Alkylensulfonatrest und
- X: ein Alkali-, Erdalkalimetall und/ oder Wasserstoff ist.

R ist bevorzugt ein linearer oder verzweigter Alkylrest mit 1 bis 30 Kohlenstoffatomen, der gegebenenfalls auch Ringe enthalten kann, ein linearer oder verzweigter Alkenylrest mit 2 bis 30 Kohlenstoffatomen, der gegebenenfalls auch Ringe enthalten kann, ein linearer oder verzweigter Hydroxyalkylrest mit 1 bis 30 Kohlenstoffatomen, ein Alkylencarboxylatrest mit 2 bis 30 Kohlenstoffatomen oder ein Alklyensulfonatrest mit 1 bis 30 Kohlenstoffatomen.

Besonders bevorzugt ist die Aminosäure ausgewählt aus der Gruppe, enthaltend Alanin, Glutaminsäure und Serin.

Handelt es sich bei der Aminosäure der Formel (1) um eine chirale Verbindung mit mindestens einem asymmetrischen Kohlenstoffatom, so kann diese in enantiomerenreiner, skalemischer oder auch racemischer Form eingesetzt werden. Als Base für die Teilneutralisation der Aminosäure vor der Umsetzung mit Ethylenoxid im ersten Verfahrensschritt können vorteilhaft Hydroxide und/ oder Carbonate von Alkali- und/ oder Erdalkalimetallen, einzeln oder als Mischung hiervon, eingesetzt werden.

Vorteilhaft ist die Verwendung von Natriumhydroxid oder Kaliumhydroxid.

Besonders vorteilhaft wird Natriumhydroxid oder Kaliumhydroxid in wässriger Lösung, insbesondere in ca. 50 Massen-%iger wässriger Lösung, eingesetzt.

Natriumhydroxid oder Kaliumhydroxid können vorteilhaft auch als Feststoff eingesetzt werden.

Vorteilhaft wird die Aminosäure mit 0,85 bis 0,99 Äquivalenten Base pro Säuregruppe deprotoniert.

Bezüglich der verwendeten Menge an Ethylenoxid ist ein Verfahren bevorzugt, bei dem 1,80 bis 2,60, besonders bevorzugt 2,00 bis 2,35 Äquivalente an Ethylenoxid pro Aminogruppe eingesetzt werden.

Auch bezüglich der Reaktionstemperatur in der ersten Stufe gibt es bevorzugte Varianten. So ist ein Verfahren bevorzugt, bei dem die Reaktionstemperatur im Bereich von 30 bis 100 °C, vorzugsweise im Bereich von 60 bis 90 °C liegt. Die Reaktionstemperatur in der ersten Verfahrensstufe schwankt während der Dosierung des Ethylenoxides vorzugsweise weniger als 40 °C, besonders bevorzugt weniger als 25 °C.

Das Verfahren kann als Batch-, Semi-Batch- oder Konti-Verfahren ausgeführt werden. Ein Verfahren, bei dem (mindestens) ein Reaktor ausgewählt aus der Gruppe, enthaltend Rührkesselreaktor, Schlaufenreaktor und Rohrreaktor, verwendet wird, ist besonders bevorzugt.
Dies ist unter Verwendung verschiedener Reaktormodelle wie Rührkesselreaktoren verschiedener Bauarten, Schlaufenreaktoren ausgeführt als Gasumlaufreaktor, Tauchstrahlreaktor, Strahldüsenreaktor oder hochbelastete Packungssäule oder Rohrreaktoren, die gasphasenfrei oder mit Gasphase betrieben werden, möglich.

Ein Verfahren, bei dem der Reaktor im Wesentlichen aus einem Material mit einem Wärmeleitfähigkeitskoeffizienten von größer 5 W/K*m besteht, ist besonders geeignet. Dabei bedeutet "im Wesentlichen", dass mehr als 50%, vorzugsweise mehr als 80% und besonders bevorzugt mehr als 90% des Reaktormaterials aus einem Material mit entsprechendem Wärmeleitfähigkeitskoeffizienten bestehen.

Als besonders geeignet erweisen sich dafür Materialien bzw. Werkstoffe wie 1.4541 (V2A-Stahl), 1.4571 (V4A-Stahl), 2.4610 (HC4-Stahl) mit einem Wärmeleitfähigkeitskoeffizienten größer 5 W/K*m, um im technischen Verfahren eine effiziente Wärmeabfuhr zu ermöglichen.

Ebenso ist ein Verfahren bevorzugt, bei dem das Lösungsmittel der ersten Stufe ausgewählt ist aus protischen Lösungsmitteln wie Wasser, Alkoholen, bevorzugt kurzkettigen Alkoholen und insbesondere Methanol, Ethanol, 2-Propanol, und/ oder polar aprotischen Lösungsmitteln wie Dimethylsulfoxid, Dimethylformamid oder N-Methylpyrrolidon. Besonders bevorzugt ist die Verwendung von Wasser als Lösungsmittel.

Die Konzentration der freien Aminosäure und ihrer Salze im Reaktionsmedium vor dem Reaktionsstart durch Beginn der Ethylenoxiddosierung kann über weite Bereiche variiert werden. Sie beträgt vorzugsweise 5 bis 80 Massen-%, besonders bevorzugt 30 bis 70 Massen-%.

Eine besonders bevorzugte Ausführungsform des Verfahrens besteht darin, dass die Aminosäure unter teilweiser oder kompletter Zugabe des Lösungsmittels mit der benötigten Menge an Base in einem separaten Reaktor oder dem für die eigentliche Ethoxylierung verwendeten Reaktor teilneutralisiert wird, bevor die Ethylenoxiddosierung gestartet wird.

Diese Verwendung zweier Reaktoren oder Behälter in Serie ist insbesondere bei einer kontinuierlichen Fahrweise vorteilhaft, um die Teilneutralisation vom ersten Verfahrensschritt, der Ethoxylierung, zu trennen. Bei einer batch-Fahrweise ist es dagegen möglich, die Base für die Teilneutralisation und das Ethylenoxid für die Umsetzung im ersten Verfahrensschritt nacheinander im gleichen Reaktor oder Behälter zuzugeben.

Ein Verfahren, bei dem das in der ersten Stufe gebildete, das Dialkanolamin enthaltende Zwischenproduktgemisch direkt dehydriert wird, stellt eine weitere bevorzugte Ausführungsform dar. Eine direkte Dehydrierung bedeutet, dass keine auf unterschiedlichen Siedepunkten basierende apparative Abtrennung von Substanzen mit Siedepunkten größer 200 °C (bei Normaldruck) zwischen der ersten und der zweiten Stufe erfolgt. Dies ist apparativ einfacher und spart somit eine Operation bei vergleichbar guter Endproduktqualität.

Die Dehydrierung erfolgt dabei unter Einsatz einer Base aus der Gruppe, enthaltend die Alkali- und Erdalkalihydroxide, bevorzugt Natriumhydroxid oder Kaliumhydroxid, wobei Natriumhydroxid besonders bevorzugt ist. Die Temperatur der zweiten Stufe liegt üblicherweise im Bereich von 140 bis 240 °C, vorzugsweise im Bereich von 150 bis 210 °C und besonders bevorzugt im Bereich von 160 bis 200 °C. Der Druck liegt üblicherweise im Bereich von Normaldruck bis 100 bar, vorzugsweise von 5 bis 50 bar und besonders bevorzugt im Bereich von 8 bis 20 bar.

Ein Verfahren, bei dem die Dehydrierung in Gegenwart eines Katalysators durchgeführt wird, dessen Haupt- und Nebenbestandteile ausgewählt sind aus den Gruppen 4 bis 12 des Periodensystems, ist dabei besonders bevorzugt, ganz besonders bevorzugt ist ein Verfahren, bei dem die Dehydrierung in Gegenwart eines Katalysators durchgeführt wird, der (mindestens) ein Metall enthält, das ausgewählt ist aus der Gruppe, enthaltend Cu, Fe, Co, Ni, Zr, Hf, Ag, Pd und Pt.

Der Katalysator kann z. B. als Pulver oder Formkörper (z. B. Extrudate, Tabletten), als Vollkontakt oder Trägerkatalysator eingesetzt werden und aus Metallen sowie Metalloxiden bestehen.

Ein Verfahren, bei dem der Gehalt an Nitrilotriessigsäure (NTA) oder deren Salze im direkten Produkt der zweiten Stufe kleiner 1 Massen% bezogen auf das Hauptprodukt ist, bildet einen weiteren Gegenstand der vorliegenden Erfindung.

Unter dem direkten Produkt der zweiten Stufe versteht man den Reaktionsaustrag, so wie er bei der oxidativen Dehydrierung anfällt. Danach kann im Falle einer Suspensionsfahrweise der Katalysator sedimentiert und/ oder abfiltriert werden. Außerdem kann anschließend noch ein gewünschter Wassergehalt eingestellt und/ oder eine Bleichung z. B. mit Wasserstoffperoxid oder UV-Licht durchgeführt werden.

Neben den Salzen (Aminopolycarboxylaten) selbst sind nach Ansäuern auch die entsprechenden Aminopolycarbonsäuren zugänglich.

Besonders bevorzugt ist dabei ein Verfahren, bei dem auch das Endprodukt, abgesehen von den vorgenannten Maßnahmen, nicht weiter aufgereinigt wird, sondern direkt in den entsprechenden Anwendungen eingesetzt wird, wie z. B. als Additiv für technische Reinigungsformulierungen für harte Oberflächen aus Metall, Kunststoff, Lack oder Glas, in alkalischen Reinigungsformulierungen für die Getränke- und Nahrungsmittelindustrie, insbesondere für die Flaschenreinigung in der Getränkeindustrie sowie für die Apparatereinigung in Molkereien, in Brauereien, in der Konserven-, der Backwaren-, der Zucker-, der fettverarbeitenden und der fleischverarbeitenden Industrie, in Geschirrreinigungsformulierungen, insbesondere in phosphatfreien Mitteln für das maschinelle Geschirrreinigen in Geschirrspülmaschinen im Haushalt oder in Gewerbebetrieben, z. B. Großküchen oder Restaurants, in Bleichbädern in der Papierindustrie, in photografischen Bleich- und Bleichfixierbädern, bei der Vorbehandlung und Bleichung in der Textilindustrie, in galvanischen Bädern zur Maskierung von verunreinigenden Schwermetallkationen, weiterhin im Bereich der Pflanzenernährung zur Behebung von Schwermetalldefiziten als Kupfer,- Eisen-, Mangan- und/ oder Zinkkomplexe. Der Einsatz ist prinzipiell überall dort vorteilhaft, wo Ausfällungen von Calcium-, Magnesium- oder Schwermetallsalzen technische Verfahren stören deshalb und verhindert werden sollen (Vermeidung von Ablagerungen und Verkrustungen in Kesseln, Rohrleitungen, Sprühdosen oder allgemein an glatten Oberflächen). Außerdem können die Aminopolycarboxylate zur Stabilisierung von Phosphaten in alkalischen Entfettungsbädern und zur Verhinderung der Ausfällung von Kalkseifen verwendet werden, um dadurch das "Anlaufen" von nicht-Eisenoberflächen zu verhindern und die Standzeit von alkalischen Reinigungsbädern zu verlängern. Daneben finden sie Anwendung in Pulver- oder Flüssigwaschmittelformulierungen für die Textilwäsche als Builder und Konservierungsmittel. In Seifen verhindern sie metallkatalysierte, oxidative Zersetzungen wie auch in Pharmazeutika, Kosmetika und Nahrungsmitteln.

Die vorliegende Erfindung wird nachfolgend durch nicht einschränkende Beispiele näher erläutert:

### Beispiel 1

Im Folgenden wurde die Aminosäure Alanin erfindungsgemäß mit 0,98 Äquivalenten Base pro Säuregruppe teilneutralisiert:

267 g (3,00 mol) Alanin wurde in 161 g Wasser suspendiert und mit 238 g (2,94 mol), d.h. 0,98 Äquivalenten Base pro Säuregruppe 49.4 Massen-%iger Natronlauge versetzt. Die resultierende Mischung wurde in einen 2.5 L-Autoklaven (Material 1.4571) gefüllt und nach entsprechender Inertisierung mit 20 bar Stickstoff beaufschlagt. Anschließend wurde 291 g (6,60 mol) Ethylenoxid bei 40-50 °C innerhalb von 6 h zudosiert und noch weitere 5 h bei dieser Temperatur nachgerührt. Nach der Entfernung der nicht umgesetzten Reste an Ethylenoxid wurde der Autoklav entleert. Auf diese Weise wurde 934 g wässriger Reaktionsaustrag als klare, farblose, viskose Lösung erhalten.

324 g (1,04 mol bezogen auf Alanin) dieses Zwischenproduktes wurde mit 199 g (2,49 mol) 50 Massen-%iger Natronlauge, 49 g Wasser und 45 g Raney-Kupfer (der Firma Evonik Degussa GmbH) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 406 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 94,0% der Theorie bezogen auf eingesetztes Alanin bestimmt. Die Ausbeute an dem entsprechenden Monoetherpolycarboxylat betrug weniger als 3% der Theorie bezogen auf eingesetztes Alanin.

Die Reaktion zum Wertprodukt erfolgte mit hoher Selektivität und hohem Umsatz, bei gleichzeitig sehr niedrigem Gehalt an unerwünschtem Monoetherpolycarboxylat. Eine Aufreinigung des Reaktionsproduktes war daher nicht erforderlich.

### Vergleichsbeispiel 1

Im Folgenden wurde die Aminosäure Alanin gemäß dem Stand der Technik mit 1,00 Äquivalenten Base pro Säuregruppe vollständig neutralisiert:

178 g (2,00 mol) Alanin wurde in 106 g Wasser suspendiert und mit 160 g (2,00 mol), d.h. 1,00 Äquivalenten Base pro Säuregruppe 50.0 Massen-%iger Natronlauge versetzt. Die resultierende Mischung wurde in einen 2.5 L-Autoklaven (Material 1.4571) gefüllt und nach entsprechender Inertisierung mit 2 bar Stickstoff beaufschlagt. Anschließend wurde 189 g (4,30 mol) Ethylenoxid bei 40-50 °C innerhalb von 4 h zudosiert und noch weitere 5 h bei dieser Temperatur nachgerührt. Nach der Entfernung der nicht umgesetzten Reste an Ethylenoxid wurde der Autoklav entleert. Auf diese Weise wurde 624 g wässriger Reaktionsaustrag als klare, farblose, viskose Lösung erhalten.

324 g (1,04 mol bezogen auf Alanin) dieses Zwischenproduktes wurde mit 208 g (2,60 mol) 50 Massen-%iger Natronlauge, 39 g Wasser und 45 g Raney-Kupfer (der Firma Evonik Degussa GmbH) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 423 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 89,9% der Theorie bezogen auf eingesetztes Alanin bestimmt. Die Ausbeute an dem entsprechenden Monoetherpolycarboxylat betrug 6% der Theorie bezogen auf eingesetztes Alanin.

Die Reaktion zum Wertprodukt erfolgte mit hohem Umsatz aber verringerter Selektivität, bei gleichzeitig erhöhtem Gehalt an unerwünschtem Monoetherpolycarboxylat. Das Reaktionsprodukt weist somit eine verringerte Reinheit auf und muss entsprechend aufgereinigt werden.

### Vergleichsbeispiel 2

Im Folgenden wurde die Aminosäure Alanin mit einem geringen Überschuss an 1,10 Äquivalenten Base pro Säuregruppe vollständig neutralisiert:

267 g (3,00 mol) Alanin wurde in 132 g Wasser suspendiert und mit 267 g (3,30 mol), d.h. 1,10 Äquivalenten Base pro Säuregruppe 49.4 Massen-%iger Natronlauge versetzt. Die resultierende Mischung wurde in einen 2.5 L-Autoklaven (Material 1.4571) gefüllt und nach entsprechender Inertisierung mit 20 bar Stickstoff beaufschlagt. Anschließend wurde 291 g (6,60 mol) Ethylenoxid bei 40-50 °C innerhalb von 6 h zudosiert und noch weitere 5 h bei dieser Temperatur nachgerührt. Nach der Entfernung der nicht umgesetzten Reste an Ethylenoxid wurde der Autoklav entleert. Auf diese Weise wurde 940 g wässriger Reaktionsaustrag als klare, farblose, viskose Lösung erhalten.

326 g (1,04 mol bezogen auf Alanin) dieses Zwischenproduktes wurde mit 190 g (2,37 mol) 50 Massen-%iger Natronlauge, 55 g Wasser und 45 g Raney-Kupfer (der Firma Evonik Degussa GmbH) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 481 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 71,5% der Theorie bezogen auf eingesetztes Alanin bestimmt. Die Ausbeute an dem entsprechenden Monoetherpolycarboxylat betrug 11% der Theorie bezogen auf eingesetztes Alanin.

Die Reaktion zum Wertprodukt erfolgte mit hohem Umsatz aber stark verringerter Selektivität, bei gleichzeitig deutlich erhöhtem Gehalt an unerwünschtem Monoetherpolycarboxylat. Das Reaktionsprodukt weist somit eine erheblich verringerte Reinheit auf und muss entsprechend aufgereinigt werden.

### Beispiel 2

Im Folgenden wurde die Aminosäure Alanin erfindungsgemäß mit ebenfalls 0,98 Äquivalenten Base pro Säuregruppe teilneutralisiert, die oxidative Dehydrierung wurde jedoch mit gegenüber Beispiel 1 verkürzter Reaktionszeit durchgeführt:

324 g (1,04 mol bezogen auf Alanin) des Zwischenproduktes aus Beispiel 1 wurde mit 200 g (2,50 mol) 50 Massen-%iger Natronlauge, 49 g Wasser und 45 g Raney-Kupfer (der Firma Evonik Degussa GmbH) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 8 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 416 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 92,5% der Theorie bezogen auf eingesetztes Alanin bestimmt. Die Ausbeute an dem entsprechenden Monoetherpolycarboxylat betrug weniger als 3% der Theorie bezogen auf eingesetztes Alanin.

Die Reaktion zum Wertprodukt erfolgte erneut mit hoher Selektivität und hohem Umsatz, bei gleichzeitig sehr niedrigem Gehalt an unerwünschtem Monoetherpolycarboxylat. Eine Aufreinigung des Reaktionsproduktes war daher wiederum nicht erforderlich.

### Beispiel 3

Im Folgenden wurde die Teilneutralisation der Aminosäure Alanin erfindungsgemäß mit lediglich 0,90 Äquivalenten Base pro Säuregruppe durchgeführt:

178 g (2,00 mol) Alanin wurde in 120 g Wasser suspendiert und mit 146 g (1,80 mol), d.h. 0,90 Äquivalenten Base pro Säuregruppe 49.4 Massen-%iger Natronlauge versetzt. Die resultierende Mischung wurde in einen 2.5 L-Autoklaven (Material 1.4571) gefüllt und nach entsprechender Inertisierung mit 20 bar Stickstoff beaufschlagt. Anschließend wurde 194 g (4,40 mol) Ethylenoxid bei 40-50 °C innerhalb von 4 h zudosiert und noch weitere 5 h bei dieser Temperatur nachgerührt. Nach der Entfernung der nicht umgesetzten Reste an Ethylenoxid wurde der Autoklav entleert. Auf diese Weise wurde 619 g wässriger Reaktionsaustrag als klare, farblose, viskose Lösung erhalten.

322 g (1,04 mol bezogen auf Alanin) dieses Zwischenproduktes wurde mit 217 g (2,71 mol) 50 Massen-%iger Natronlauge, 38 g Wasser und 45 g Raney-Kupfer (der Firma Evonik Degussa GmbH) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 425 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 94,1% der Theorie bezogen auf eingesetztes Alanin bestimmt. Die Ausbeute an dem entsprechenden Monoetherpolycarboxylat betrug weniger als 3% der Theorie bezogen auf eingesetztes Alanin.

Die Reaktion zum Wertprodukt erfolgte erneut mit hoher Selektivität und hohem Umsatz, bei gleichzeitig sehr niedrigem Gehalt an unerwünschtem Monoetherpolycarboxylat. Eine Aufreinigung des Reaktionsproduktes war daher wiederum nicht erforderlich.

### Beispiel 4

Im Folgenden wurde die Teilneutralisation der Aminosäure Alanin erfindungsgemäß mit ebenfalls lediglich 0,90 Äquivalenten Base pro Säuregruppe durchgeführt, die Ethoxylierung jedoch unter gegenüber Beispiel 3 veränderten Verfahrensparametern durchgeführt:

178 g (2,00 mol) Alanin wurde in 46 g Wasser suspendiert und mit 146 g (1,80 mol), d.h. 0,90 Äquivalenten Base pro Säuregruppe 49.4 Massen-%iger Natronlauge versetzt. Die resultierende Mischung wurde in einen 2.5 L-Autoklaven (Material 1.4571) gefüllt und nach entsprechender Inertisierung mit 20 bar Stickstoff beaufschlagt. Anschließend wurde 203 g (4,60 mol) Ethylenoxid bei 80-85 °C innerhalb von 2 h zudosiert und noch weitere 2 h bei dieser Temperatur nachgerührt. Nach der Entfernung der nicht umgesetzten Reste an Ethylenoxid wurde der Autoklav entleert. Auf diese Weise wurde 527 g wässriger Reaktionsaustrag als klare, farblose, viskose Lösung erhalten.

274 g (1,04 mol bezogen auf Alanin) dieses Zwischenproduktes wurde mit 217 g (2,71 mol) 50 Massen-%iger Natronlauge, 81 g Wasser und 45 g Raney-Kupfer (der Firma Evonik Degussa GmbH) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 403 g Wasser verdünnt und dann entleert. Das Produkt wurde als klare, farblose, viskose Lösung erhalten. Mittels HPLC wurde eine Ausbeute (= Selektivität * Umsatz) an Methylglycin-N,N-diessigsäure Trinatriumsalz (MGDA-Na₃) von 94,2% der Theorie bezogen auf eingesetztes Alanin bestimmt. Die Ausbeute an dem entsprechenden Monoetherpolycarboxylat betrug weniger als 3% der Theorie bezogen auf eingesetztes Alanin.

Die Reaktion zum Wertprodukt erfolgte erneut mit hoher Selektivität und hohem Umsatz, bei gleichzeitig sehr niedrigem Gehalt an unerwünschtem Monoetherpolycarboxylat. Eine Aufreinigung des Reaktionsproduktes war daher wiederum nicht erforderlich.

## Patentansprüche

1. Verfahren zur Herstellung von Aminopolycarboxylaten ausgehend von einer Aminosäure, die in einem
- ersten Verfahrensschritt mit Ethylenoxid zu einem Zwischenproduktgemisch, enthaltend das entsprechende Dialkanolamin, umgesetzt wird, und danach das Zwischenproduktgemisch in einem
- zweiten Verfahrensschritt katalytisch unter Einsatz einer Base zum entsprechenden Aminopolycarboxylat umgesetzt wird, **dadurch gekennzeichnet, dass**
- die Aminosäure vor der Umsetzung mit Ethylenoxid im ersten Verfahrensschritt einer Teilneutralisation mit 0,70 bis 0,99 Äquivalenten Base pro Säuregruppe zugeführt wird oder dass im ersten Verfahrensschritt eine bereits mit 0,70 bis 0,99 Äquivalenten Base pro Säuregruppe teilneutralisierte Aminosäure eingesetzt wird.

2. Verfahren nach Anspruch 1, **dadurch gekennzeichnet, dass** die Aminosäure ausgewählt ist aus der Gruppe der Aminosäuren der Formel (1) 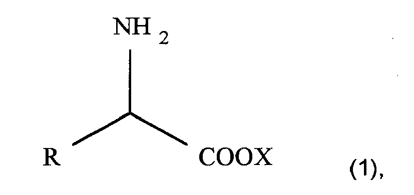 wobei
R ein Alkyl-, Alkenyl-, Alkinyl-, Aryl-, Aralkyl-, Hydroxyalkyl-, Hydroxyaralkyl-, Alkylencarboxyl- oder ein Alkylensulfonatrest und
X ein Alkali-, Erdalkalimetall und/oder Wasserstoff ist.

3. Verfahren nach Anspruch 2, **dadurch gekennzeichnet, dass** R ein linearer oder verzweigter Alkylrest mit 1 bis 30 Kohlenstoffatomen, der gegebenenfalls auch Ringe enthalten kann, ein linearer oder verzweigter Alkenylrest mit 2 bis 30 Kohlenstoffatomen, der gegebenenfalls auch Ringe enthalten kann, ein linearer oder verzweigter Hydroxyalkylrest mit 1 bis 30 Kohlenstoffatomen, ein Alkylencarboxylatrest mit 2 bis 30 Kohlenstoffatomen oder ein Alklyensulfonatrest mit 1 bis 30 Kohlenstoffatomen ist.

4. Verfahren nach Anspruch 3, **dadurch gekennzeichnet, dass** die Aminosäure ausgewählt ist aus der Gruppe, enthaltend Alanin, Glutaminsäure und Serin.

5. Verfahren nach einem der Ansprüche 1 bis 4, **dadurch gekennzeichnet, dass** die zur Teilneutralisation der Aminosäure eingesetzte Base ein Hydroxid und/ oder ein Carbonat eines Alkali- und/ oder eines Erdalkalimetalles, oder eine Mischung hiervon ist.

6. Verfahren nach Anspruch 5, **dadurch gekennzeichnet, dass** die zur Teilneutralisation der Aminosäure eingesetzte Base Natriumhydroxid oder Kaliumhydroxid ist.

7. Verfahren nach Anspruch 6, **dadurch gekennzeichnet, dass** das Natriumhydroxid oder Kaliumhydroxid in wässriger Lösung, bevorzugt in 50 Massen-%iger wässriger Lösung, eingesetzt wird.

8. Verfahren nach einem der Ansprüche 1 bis 7, **dadurch gekennzeichnet, dass** der erste Verfahrensschritt bei einer Reaktionstemperatur im Bereich von 30 bis 100 °C, bevorzugt bei einer Reaktionstemperatur im Bereich von 60 bis 90 °C, durchgeführt wird.

9. Verfahren nach einem der Ansprüche 1 bis 8, **dadurch gekennzeichnet, dass** der erste Verfahrensschritt dergestalt durchgeführt wird, dass die Reaktionstemperatur während des ersten Verfahrensschrittes um weniger als 40 °C, bevorzugt um weniger als 25 °C schwankt.

10. Verfahren nach einem der Ansprüche 1 bis 9, **dadurch gekennzeichnet, dass** der erste Verfahrensschritt in Gegenwart eines protischen und/oder polar aprotischen Lösungsmittels, bevorzugt in Gegenwart von Wasser, durchgeführt wird.

## Claims

1. A process for preparing aminopolycarboxylates proceeding from an amino acid which, in a
- first process step is reacted with ethylene oxide to give an intermediate mixture comprising the corresponding dialkanolamine, and then the intermediate mixture, in a
- second process step is converted catalytically using a base to the corresponding aminopolycarboxylate, wherein
- the amino acid, before the reaction with ethylene oxide in the first process step, is supplied to a partial neutralization with 0.70 to 0.99 equivalent of base per acid group, or, in the first process step, an amino acid which has already been partly neutralized with 0.70 to 0.99 equivalent of base per acid group is used.

2. The process according to claim 1, wherein the amino acid is selected from the group of the amino acids of the formula (1) 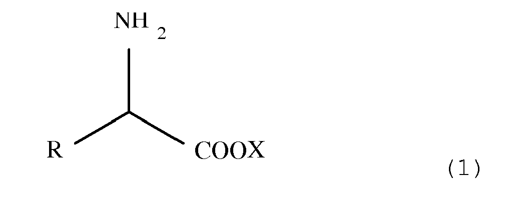 where
R is an alkyl, alkenyl, alkynyl, aryl, aralkyl, hydroxyalkyl, hydroxyaralkyl, alkylenecarboxyl or alkylenesulfonate radical and
X is an alkali metal, alkaline earth metal and/or hydrogen.

3. The process according to claim 2, wherein R is a linear or branched alkyl radical which has 1 to 30 carbon atoms and may optionally also comprise rings, a linear or branched alkenyl radical which has 2 to 30 carbon atoms and may optionally also comprise rings, a linear or branched hydroxyalkyl radical having 1 to 30 carbon atoms, an alkylenecarboxylate radical having 2 to 30 carbon atoms or an alklyenesulfonate radical having 1 to 30 carbon atoms.

4. The process according to claim 3, wherein the amino acid is selected from the group comprising alanine, glutamic acid and serine.

5. The process according to any of claims 1 to 4, wherein the base used for partial neutralization of the amino acid is a hydroxide and/or a carbonate of an alkali metal and/or of an alkaline earth metal, or a mixture thereof.

6. The process according to claim 5, wherein the base used for partial neutralization of the amino acid is sodium hydroxide or potassium hydroxide.

7. The process according to claim 6, wherein the sodium hydroxide or potassium hydroxide is used in aqueous solution, preferably in 50% by mass aqueous solution.

8. The process according to any of claims 1 to 7, wherein the first process step is performed at the reaction temperature in the range from 30 to 100°C, preferably at a reaction temperature in the range from 60 to 90°C.

9. The process according to any of claims 1 to 8, wherein the first process step is performed in such a way that the reaction temperature during the first process step varies by less than 40°C, preferably by less than 25°C.

10. The process according to any of claims 1 to 9, wherein the first process step is performed in the presence of a protic and/or polar aprotic solvent, preferably in the presence of water.

## Revendications

1. Procédé de fabrication d'aminopolycarboxylates à partir d'un acide aminé, qui
- lors d'une première étape de procédé, est mis en réaction avec de l'oxyde d'éthylène pour former un mélange de produits intermédiaires, contenant la dialcanolamine correspondante, puis le mélange de produits intermédiaire,
- lors d'une seconde étape de procédé, est mis en réaction catalytiquement en utilisant une base pour former l'aminopolycarboxylate correspondant, **caractérisé en ce que**
- l'acide aminé est introduit avant la mise en réaction avec l'oxyde d'éthylène lors de la première étape de procédé dans une neutralisation partielle avec 0,70 à 0,99 équivalent de base par groupe acide, ou **en ce que**, lors de la première étape de procédé, un acide aminé déjà partiellement neutralisé avec 0,70 à 0,99 équivalent de base par groupe acide est utilisé.

2. Procédé selon la revendication 1, **caractérisé en ce que** l'acide aminé est choisi dans le groupe constitué par les acides aminés de formule (1) 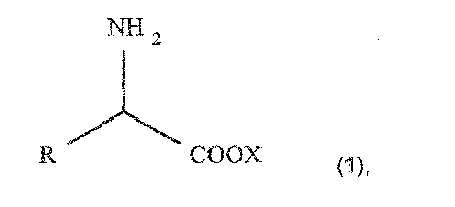 dans laquelle
R représente un radical alkyle, alcényle, alcynyle, aryle, aralkyle, hydroxyalkyle, hydroxyaralkyle, carboxylate d'alkylène ou sulfonate d'alkylène, et
X représente un métal alcalin, alcalino-terreux et/ou l'hydrogène.

3. Procédé selon la revendication 2, **caractérisé en ce que** R représente un radical alkyle linéaire ou ramifié de 1 à 30 atomes de carbone, qui peut éventuellement également contenir des cycles, un radical alcényle linéaire ou ramifié de 2 à 30 atomes de carbone, qui peut éventuellement également contenir des cycles, un radical hydroxyalkyle linéaire ou ramifié de 1 à 30 atomes de carbone, un radical carboxylate d'alkylène de 2 à 30 atomes de carbone ou un radical sulfonate d'alkylène de 1 à 30 atomes de carbone.

4. Procédé selon la revendication 3, **caractérisé en ce que** l'acide aminé est choisi dans le groupe contenant l'alanine, l'acide glutamique et la sérine.

5. Procédé selon l'une quelconque des revendications 1 à 4, **caractérisé en ce que** la base utilisée pour la neutralisation partielle de l'acide aminé est un hydroxyde et/ou un carbonate d'un métal alcalin et/ou alcalino-terreux, ou un mélange de ceux-ci.

6. Procédé selon la revendication 5, **caractérisé en ce que** la base utilisée pour la neutralisation partielle de l'acide aminé est l'hydroxyde de sodium ou l'hydroxyde de potassium.

7. Procédé selon la revendication 6, **caractérisé en ce que** l'hydroxyde de sodium ou l'hydroxyde de potassium est utilisé en solution aqueuse, de préférence en solution aqueuse à 50 % en masse.

8. Procédé selon l'une quelconque des revendications 1 à 7, **caractérisé en ce que** la première étape de procédé est réalisée à une température de réaction dans la plage allant de 30 à 100 °C, de préférence à une température de réaction dans la plage allant de 60 à 90 °C.

9. Procédé selon l'une quelconque des revendications 1 à 8, **caractérisé en ce que** la première étape de procédé est réalisé de sorte que la température de réaction pendant la première étape de procédé varie de moins de 40 °C, de préférence de moins de 25 °C.

10. Procédé selon l'une quelconque des revendications 1 à 9, **caractérisé en ce que** la première étape de procédé est réalisée en présence d'un solvant protique et/ou aprotique polaire, de préférence en présence d'eau.
